# EUROPEAN PATENT APPLICATION

(11) **EP 4 835 081 A1**
(43) Date of publication of application: **04.11.2026**
(21) Application number: 24909804.7
(22) Date of filing: 11.08.2024
(51) Int. Cl.: C12N 15/31, C12N 15/52, C12N 15/85, C12N 15/113, A61K 48/00, A61K 31/713

(54) **VECTOR FOR PREPARING CIRCULAR RNA AND CONSTRUCTION METHOD**

(30) Priority: 26.12.2023 CN 202311808436
(71) Applicant: Shenzhen Genturn Life Co., Ltd., Shenzhen, Guangdong 518000 (CN)
(72) Inventor: TANG, Longqing, Shenzhen, Guangdong 518000 (CN); WANG, Binxu, Shenzhen, Guangdong 518000 (CN); ZHOU, Xiangjun, Shenzhen, Guangdong 518000 (CN)
(74) Representative: ABG Intellectual Property Law, S.L.
(86) International application number: PCT/CN2024/111319
(87) International publication number: WO 2025/138852

(57) **Abstract**

A flexible method for constructing a vector for preparing circular RNA without being limited by substrate sequence is provided. The method comprises following steps: determining a target site sequence NNUNNNN to be cyclized, and dividing said sequence into E1: NNU and E2: NNNN; using the E1 and E2 sequences as references to design IGS sequences to form complementary pairings with E1 and E2, respectively; adding 5' and 3' homologous arms, as well as IRES and CDS and other elements, respectively. In the provided method, circular RNA can be prepared for any target sequence without residual sequences, and the described method has high cyclization efficiency. Also provided is a FlexCirc circularization system designed on the basis of an Azoarcus group I intron ribozyme, which can form circular RNA. The circularization substrate sequence has the characteristics of flexible design, and the circularization efficiency of achieving a high proportion of circularization

## Description

### Technical Field

The present invention belongs to the field of molecular biology and bioengineering technology, and specifically relates to recombinant nucleic acids and vectors for preparing circular RNA, and methods for preparing circular RNA.

### Background Art

Circular RNA, as the name suggests, is a class of RNA molecules with a circular closed structure, which are widely present in nature. Circular RNA can generally be formed in different organisms through a back-splicing mechanism. Compared with linear mRNA, circular RNA lacks 5' and 3' ends, and therefore can resist hydrolysis by exonucleases and exhibits relatively higher stability. In 2018, Wesselhoeft R.A. published an article entitled "Engineering circular RNA for potent and stable translation in eukaryotic cells" in Nature Communications, pioneering the application of circular RNA for protein expression. The article utilized a microbially derived Group I ribozyme, namely the Anabaena group I intron, which is divided to form a 5' half intron and a 3' half intron, and rearranged to form the PIE (permuted intron-exon) basic structure. Based on the PIE basic structure, flanking homology arms and internal spacer sequences are added, thereby improving the circularization efficiency and cargo size of the Anabaena group I intron PIE, and so this circularization system is defined as AnaPIE (Wesselhoeft, Kowalski et al. 2018). However, for the AnaPIE circularization system, approximately 186 bp of bases would remain in the circular RNA as a scar sequence, and this residual scar sequence has been shown to have potential immunogenicity issues (Liu, Guo et al. 2022).

In 2021, Rausch studied Tetrahymena T4td ribozyme and identified the substrate core sequence of the Tetrahymena T4td ribozyme as a 10 bp core sequence (Rausch, Heinz et al. 2021). To address the issue of residual scars in circular RNA, the patent publication CN114574483A, based on Rausch's 2021 research, further reduced the core substrate sequence to 8 bp: TTGGGT CT. This invention hides the sequence TTGGGT CT within the sequence to be circularized, such as in the target protein CDS or within the IRES element, and used the PIE structure to design the CleanPIE circularization system with scar sequences hidden. However, the circularization site of this CleanPIE system is still limited by the above-mentioned substrate core sequence TTGGGT CT, and cannot be circularized at arbitrary sites.

Patent KR102442946B1 discloses another circularization system, the designed STS system, utilizing the Tetrahymena T4td ribozyme, where the circularization site is formed by a G:U wobble base pair between the 5' IGS and the 3' target site, with G located within the 5' IGS and U located at the terminus of the 3' target site region. This STS circularization system does not require dividing the ribozyme; it only requires maintaining complementary pairing between the 5' IGS sequence and the 3' target site sequence to form circular RNA. However, according to the circularization efficiency disclosed in patent KR102442946B1, the proportion of circular RNA is only 12%, which is insufficient for effective production and application.

### Summary of the Invention

### Basic Definitions

In the claims and/or specification, when used in conjunction with the term "comprising", the words "a" or "an" may refer to "one" but may also refer to "one or more", "at least one" and "one or more than one".

As used in the claims and specification, the words "including", "having", "comprising" or "containing" indicate the presence of the stated features, integers, steps, operations, elements, or components, but do not exclude the presence or addition of one or more other features, integers, steps, operations, elements, components, or groups thereof.

Throughout the application, the term "about" means: a value includes the standard deviation of the error of the apparatus or method used to determine that value.

The terms "polypeptide", "peptide" and "protein" are used interchangeably herein and are amino acid polymers of any length. The polymer may be linear or branched, may comprise modified amino acids, and may be interrupted by non-amino acids. The term also encompasses amino acid polymers that have been modified (e.g., disulfide bond formation, glycosylation, lipidation, acetylation, phosphorylation, or any other operations, such as conjugation with a labeling component).

### Definitions of Technical Terms

### Homology Arms (5' arm, 3' arm)

The 5' and 3' homology arms (5' arm, 3' arm) may be synthetic sequences that differ from internal homologous regions but have similar functions. The length of the homology arms may be, for example, about 5-55 nucleotides, about 9-19 nucleotides, for example, a length of about 5, about 10, about 20, about 30, about 40, or about 50 nucleotides. In another embodiment, the length of the homology arms may be 9 nucleotides. In another embodiment, the length of the homology arms may be 19 nucleotides. In certain embodiments, the length of the homology arms is at least 5, 6, 7, 8, 9, 10, 11, 12, 13, 14, 15, 16, 17, 18, or 19 nucleotides. In certain embodiments, the length of the homology arms does not exceed 50, 45, 40, 35, 30, 25, or 20 nucleotides. In certain embodiments, the length of the homology arms is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, or 55 nucleotides.

### Ribozyme

A "ribozyme" is used to describe an RNA molecule with catalytic activity, typically having a specific three-dimensional structure. In some embodiments, the ribozyme recognition site of the present invention refers to a polynucleotide sequence that can internally undergo phosphodiester bond cleavage and religation when the RNA molecule forms a three-dimensional structural ribozyme molecule with catalytic function.

### PIE System

Also known as permuted introns and exons, it refers to a method in which a Group I intron is divided at a specific position to form a 5' half intron and a 3' half intron, the two divided intron parts are rearranged, and a target gene sequence is added in the middle of the rearranged introns. Using the self-splicing and ligation reactions of the Group I Intron, the target gene sequence is ligated to form a circular RNA molecule.

### Spacer

As described herein, a spacer may refer to a domain that provides space between other domains. Relative to a comparable engineered polynucleotide lacking a spacer domain, the addition of a spacer may provide improvement (e.g., increased specificity, enhanced editing efficiency, etc.) of the engineered polynucleotide over a target polynucleotide. In some cases, a spacer domain may comprise a filler sequence. In some cases, the filler sequence may comprise a spacer domain.

In some cases, as used herein, a "space" refers to any continuous nucleotide sequence that: 1) is predicted to avoid interfering with proximal structures, such as from IRES, coding or non-coding regions, or introns, 2) is at least 7 nucleotides in length (optionally not exceeding 100 nucleotides), 3) is located downstream and close to the 3' intron fragment and/or upstream and close to the 5' intron fragment, and/or 4) comprises one or more of the following: a) an unstructured region of at least 5 nt in length, b) a region predicted to base-pair with a distal (i.e., non-adjacent) sequence of at least 5 nt in length, which includes another spacer, and/or c) a structured region of at least 7 nt in length, limited to the sequence of the spacer.

In certain embodiments, the spacer sequence may be, for example, at least 10 nucleotides in length, at least 15 nucleotides in length, or at least 30 nucleotides in length. In certain embodiments, the length of the spacer sequence is at least 7, 8, 9, 10, 11, 12, 13, 14, 15, 16, 17, 18, 19, 20, 25, or 30 nucleotides. In certain embodiments, the length of the spacer sequence does not exceed 100, 90, 80, 70, 60, 50, 45, 40, 35, or 30 nucleotides.

The spacer sequence may be a polyA sequence, a polyA-C sequence, a polyC sequence, or a poly-U sequence, or may be specifically modified according to the IRES. The spacer sequences described herein may have two functions: (1) promoting circularization and (2) promoting functionality by allowing the intron and IRES to fold correctly. More specifically, the spacer sequences described herein are designed with three priorities: 1) being inert to the folding of proximal intron and IRES structures; 2) sufficiently separating the intron and IRES secondary structures; and 3) comprising a spacer-spacer complementary region to facilitate the formation of a "splicing bubble". In one embodiment, the vector is compatible with many possible IRES and coding or non-coding regions, as well as two spacer sequences.

The term "Flexible circularization (FlexCirc) system" may be interpreted as a pliable circularization system or elastic circularization system. As opposed to the disadvantage of the AnaPIE circularization system that forms a residual scar sequence in the final circular RNA product, the FlexCirc circularization system of the present invention does not retain any scar sequence in the final circular RNA product. As opposed to the CleanPIE circularization system that is limited by a fixed substrate sequence, the FlexCirc circularization system of the present invention is not limited by the substrate sequence. The FlexCirc circularization system can first determine the sequences of the sites E1 and E2 to be circularized, and by mutating the IGS sequence to form complementary pairings with E1 and E2, maintain the stability of the substrate domain and preserve the ability of the circularization reaction. The E1 and E2 sequences retained in the final circular RNA product can, according to the target site sequences to be circularized, have the technical characteristics of being flexible, variable, and freely designable.

### Definitions of Genetic Engineering Terms

The term "circular" as used in the context of a nucleic acid molecule may generally refer to a nucleic acid molecule representing a polynucleotide sequence in a circular two-dimensional form, with one nucleotide ligated to another, wherein the represented polynucleotide is circular.

A promoter refers to an initiation control region sequence that can be used to drive the expression of an associated coding region in a desired host cell, and is familiar to those skilled in the art. In practice, any promoter capable of driving these genetic elements is suitable for use in the present disclosure. The termination control region may also be derived from various genes native to the preferred host.

The term "coding region" refers to a nucleic acid molecule sequence capable of being translated into amino acids and ultimately forming a protein. More specifically, it refers to a nucleic acid molecule sequence in a messenger RNA molecule that can be translated into amino acids and ultimately form a protein.

The term "recombinant nucleic acid molecule" refers to a polynucleotide having sequences that are not ligated together in nature. The recombinant polynucleotide may be included in a suitable vector, and the vector may be used to transform into a suitable host cell. The polynucleotide is then expressed in the recombinant host cell to produce, for example, a "recombinant polypeptide", "recombinant protein" , "fusion protein" etc.

The term "encoding" as applied to a polynucleotide may refer to a polynucleotide which, if in its native state or when manipulated by methods well known to those skilled in the art, can be transcribed, translated, or transcribed and translated to produce an mRNA for a polypeptide or a fragment thereof, and may be referred to as "encoding" the polypeptide. The antisense strand can be the complementary strand of such a nucleic acid, and the coding sequence can be derived therefrom.

A "coding sequence" is a nucleic acid sequence that is transcribed into RNA (such as mRNA, rRNA, tRNA, snRNA, sense RNA, or antisense RNA). In other aspects, the RNA is not translated to produce a protein, but functions as an RNA molecule to regulate protein expression.

The term "vector" may refer to a nucleic acid construct designed for transfer between different hosts, including but not limited to plasmids, viruses, cosmids, phages, BACs, YACs, etc. In some embodiments, a "viral vector" is defined as a virus or viral particle produced by recombination, comprising a polynucleotide to be delivered to a host cell in vivo, ex vivo, or in vitro. In some embodiments, the plasmid vector may be prepared from commercially available vectors. In other embodiments, the viral vector may be produced from baculovirus, retrovirus, adenovirus, AAV, etc., according to techniques known in the art. In one embodiment, the viral vector is a lentiviral vector. Examples of viral vectors include retroviral vectors, adenoviral vectors, adeno-associated viral vectors, alphaviral vectors, etc.

In some cases, the recombinant vector is a binary vector capable of functioning in multiple organisms. The organism may be a microorganism, a plant, or an animal.

The term "host cell" refers to a cell into which an exogenous polynucleotide has been introduced, including progeny of such cells. Host cells include "transformants" and "transformed cells", which include primarily transformed cells and progeny derived therefrom. Host cells can be any type of cell system used to produce the protein molecules of the present invention, including eukaryotic cells, such as mammalian cells, insect cells, yeast cells; and prokaryotic cells, such as E.coli cells. Host cells include cultured cells, as well as cells within transgenic animals, transgenic plants, or cultured plant tissues or animal tissues.

In the present disclosure, the term "recombinant cell" generally refers to a cell with stably inherited unique traits, obtained by modifying or recombining genetic materials of a host cell using genetic engineering techniques or cell fusion techniques, encompassing host cells that differ from the parental cells after introduction of a recombinant nucleic acid molecule, a recombinant expression vector, or a circular RNA, and may be eukaryotic or prokaryotic cells. The terms "transformed" or "transfected" as used herein refer to the introduction of a nucleic acid (such as a vector) into a cell by various techniques known in the art. Suitable host cells may be transformed or transfected with the nucleic acids of the present invention, such as DNA and/or RNA sequences, and may be used for the expression and/or secretion of the target protein. Examples of suitable host cells that may be used in the present invention include immortalized hybridoma cells, NS/0 myeloma cells, HEK293T, HEK293, 293 cells, Chinese hamster ovary (CHO) cells, HeLa cells, Cap cells (human amniotic fluid-derived cells), CoS cells, 3T3, NS0, HT-1080, PERC6, CAP, HKB-11, Huh-7.

The terms "transformation, transfection, transduction" have meanings generally understood by those skilled in the art, i.e., the process of introducing exogenous nucleic acids (DNA, RNA, etc.) into a host. The methods of transformation, transfection, and transduction include any method of introducing a nucleic acid into a cell, including but not limited to electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method.

"Complementary" refers to the ability of nucleic acids to form hydrogen bonds through conventional Watson-Crick base pairing or other non-conventional types of base pairing. For the nucleic acid molecules of the present invention, the binding free energy of a nucleic acid molecule with its complementary sequence is sufficient to allow the relevant function of the nucleic acid to proceed, such as RNAi activity. The determination of the binding free energy of nucleic acid molecules is well known in the art (see, e.g., Turner et al., 1987, CSH Symp. Quant. Biol., 1987, LII, pp. 123-133; Frier et al., P.N.A.S., 1986, 83, 9373-9377; Turner et al., J Am. Chem. Soc, 1987, 109, 3783-3785). The percentage of complementarity refers to the number of nucleotides in a nucleic acid molecule that can base pair with a second nucleic acid sequence (e.g., 5, 6, 7, 8, 9, or 10 nucleotides out of a total of 10 nucleotides in a first oligonucleotide are base-paired with the second nucleic acid sequence having 10 nucleotides, representing 50%, 60%, 70%, 80%, 90%, and 100% complementarity, respectively). "Fully complementary" means that all continuous residues of a nucleic acid sequence will form hydrogen bonds with the same number of continuous residues in the second nucleic acid sequence.

### Treatment-Related Definitions

The terms "individual", "patient" or "subject" include (selected and added depending on whether there is a therapeutic use) mammals. Mammals include, but are not limited to, domestic animals (e.g., cattle, sheep, cats, dogs, and horses), primates (e.g., humans and non-human primates such as monkeys), rabbits, and rodents (e.g., mice and rats).

The term "treatment" as used herein may be used to mean obtaining a desired pharmacological effect, physiological effect, or any combination thereof. In some cases, treatment may reverse side effects attributable to a disease or condition. In some cases, treatment may stabilize a disease or condition. In some cases, treatment may delay the progression of a disease or condition. In some cases, treatment may cause regression of a disease or condition. In some cases, treatment may prevent the occurrence of a disease or condition. In some embodiments, the therapeutic effect may be measurable. In some cases, measurements may be compared before and after administration of the composition. In some cases, treatment includes, after suffering from a disease in a suject, contacting (e.g., administering) the subject with the circular RNA, circularized precursor RNA, and the recombinant nucleic acid vector, composition, etc. of the present invention, thereby alleviating the symptoms of the disease compared to when not contacted, which does not mean that the symptoms of the disease must be completely inhibited. Suffering from a disease means: the body has developed symptoms of the disease.

The term "prevent" refers to: before suffering from a disease in a subject, contacting (e.g., administering) the subject with the circular RNA, recombinant nucleic acid vector, composition, etc. of the present invention, thereby alleviating symptoms after suffering from the disease compared to when not contacted, which does not mean that the disease must be completely prevented.

The term "effective amount" refers to an amount or dose of the recombinant nucleic acid molecule, recombinant expression vector, circularized precursor RNA, circular RNA, vaccine, or composition of the present invention that, when administered to a patient in a single or multiple doses, produces the desired effect in a patient in need of treatment or prevention. The effective amount can be readily determined by the attending physician as those skilled in the art by considering various factors, such as: the species of mammal; its size, age, and general health; the specific disease involved; the degree or severity of the disease; the response of the individual patient; the specific antibody administered; the mode of administration; the bioavailability characteristics of the administered formulation; the selected dosing regimen; and the use of any concomitant therapy.

### Variant Verification

To further verify the technical effects, the inventors conducted additional verification of multiple variants of exons, spacer sequences, introns, etc., based on the sequences disclosed in the present invention. With at least 66%, 67%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9% identity, verifications are performed for various types of anticodon stems, introns from different sources, spacers of different lengths, etc. By truncation, mutation, dividing, and other methods to ensure homology of at least 66%, and by employing the verification methods described in the following description and specific embodiments-synthesizing recombinant nucleic acid molecules first, then performing circularization reactions, followed by designing PCR primers for specific sites-the inventors verified circularization effects and confirmed that the same technical effects can be achieved for these variants.

The verification method for the variants may employ comparing a new sequence with a query sequence to determine the percentage of sequence identity. Standard methods commonly used for comparing the similarity and amino acid positions of two nucleic acids or polypeptides may be used for such comparison. Computer programs, such as BLAST or FASTA, for example, may be used to align two polypeptides for optimal amino acid alignment (along the full length of one or both sequences, or along predetermined local portion of one or both sequences). Such programs provide "gap" opening penalties and "gap" extension penalties, and a scoring matrix such as PAM250 (a standard scoring matrix; see Dayhoff et al., Atlas of Protein Sequence and Structure, Vol. 5, Supp. 3 (1978)) or a BLOSUM scoring matrix may be used in combination with the computer program. The percentage of identity is then calculated.

To improve the technical effects of the coding region, the inventors performed sequence improvements on fluorescent proteins in various structures, including but not limited to codon optimization techniques for enhancing the luminescence effect of fluorescent proteins.

Existing circularization systems based on Group I ribozymes are limited by the limitation of substrate sequences, cannot circularize arbitrary sequences, and will form residual sequences, leading to potential immunogenicity issues.

The present invention discloses a method for constructing a vector for circular RNA preparation, which can achieve circular RNA preparation for arbitrary sequences without substrate sequence limitations, without residual redundant sequences, and with high circularization efficiency.

To solve the above technical problems, the present invention employs the following technical solutions.

The present invention discloses a flexible circularization (FlexCirc) system, the system comprises: a 5' homology arm, an IGS sequence, a ribozyme, a substrate E2 sequence, a spacer sequence, an IRES element, a target protein CDS sequence, a substrate E1 sequence, and a 3' homology arm.

Preferably, the circularization system is a recombinant nucleic acid molecule.

Preferably, the system comprises, in sequence from the 5' end to the 3' end, the following elements: the 5' homology arm, the IGS sequence, the ribozyme, the substrate E2 sequence, the spacer sequence, the IRES element, the target protein CDS sequence, the substrate E1 sequence, and the 3' homology arm.

Preferably, the system further comprises a T7 promoter sequence at the 5' end, and a DNA linearization enzyme cleavage site.

Preferably, the enzyme cleavage site is EcoR I.

Preferably, the substrate E1 sequence is selected from SEQ ID NO: 8, SEQ ID NO: 9, or SEQ ID NO: 12; and the substrate E2 sequence is selected from SEQ ID NO: 13, SEQ ID NO: 20, or SEQ ID NO: 21.

Preferably, a sequence of the 5' homology arm is as shown in SEQ ID NO: 1.

Preferably, the IGS sequence is selected from SEQ ID NO: 5, SEQ ID NO: 6, or SEQ ID NO: 7.

Preferably, the ribozyme sequence is as shown in SEQ ID NO: 22.

Preferably, the spacer sequence is selected from SEQ ID NO: 23 or SEQ ID NO: 26.

Preferably, the IRES element sequence is as shown in SEQ ID NO: 24.

Preferably, the target protein CDS sequence is as shown in SEQ ID NO: 25.

Preferably, a sequence of the 3' homology arm is selected from SEQ ID NO: 27, SEQ ID NO: 28, or SEQ ID NO: 29.

Preferably, the circularization system is a recombinant nucleic acid molecule, and the sequence of the recombinant nucleic acid molecule is as shown in SEQ ID NO: 17, SEQ ID NO: 18, or SEQ ID NO: 19.

The present invention discloses a recombinant vector, the recombinant vector comprises the flexible circularization system.

Preferably, the recombinant vector is pBlueScript, but this is not intended to be limiting; any recombinant vector capable of achieving the invention in the prior art is within the scope of protection.

The present invention discloses a cell, wherein the cell comprises the flexible circularization system or the recombinant vector; the cell may also be referred to as a recombinant cell.

The present invention discloses a method for preparing circular RNA, the method comprises transcribing a precursor RNA using the recombinant vector, and further preparing the circular RNA from the precursor RNA.

The present invention discloses a composition, characterized in that the composition comprises at least one of the flexible circularization system, the recombinant vector, and the cell of the present invention.

The present invention discloses a design method for a flexible circularization (FlexCirc) system, characterized by comprising the following steps:
1) Determining a site sequence to be circularized, NNUNNNN, and dividing it into E1: NNU and E2: NNNN, wherein N is any nucleotide;
2) Using the E1 and E2 sequences as a reference, designing an IGS sequence to form base complementary pairings with E1 and E2, respectively;
3) Further adding 5' and 3' homology arm sequences, a ribozyme sequence, IRES, a spacer sequence, and a target protein CDS sequence, respectively.

Preferably, in step 3), the adding sequence of each element from the 5' end to the 3' end is: the 5' homology arm, the IGS sequence, the ribozyme, the substrate E2 sequence, the spacer sequence, the IRES element, the target protein CDS sequence, the substrate E1 sequence, and the 3' homology arm.

Preferably, characterized in that, the method further comprises:
4) Introducing a T7 promoter sequence at the 5' end and a DNA template linearization enzyme cleavage site into the flexible circularization system.

Preferably, the enzyme cleavage site is EcoR I, but this is not intended to be limiting; any enzyme cleavage site capable of achieving enzymatic cleavage is within the scope of protection.

The present invention discloses a recombinant nucleic acid molecule, characterized in that the recombinant nucleic acid molecule comprises: a 5' homology arm, a first intron, a substrate enhancement element, a spacer sequence, a translation initiation element, a coding/non-coding region, a spacer sequence, a substrate enhancement element, a second intron, and a 3' homology arm.

Preferably, the recombinant nucleic acid molecule comprises, in sequence from the 5' end to the 3' end: the 5' homology arm, the first intron, the substrate enhancement element, the spacer sequence, the translation initiation element, the coding/non-coding region, the spacer sequence, the substrate enhancement element, the second intron, and the 3' homology arm.

Preferably, the intron fragment is preferably a Group I intron fragment, more preferably an Azoarcus group I intron; further, the Group I intron may be derived from any one of the following Group I introns: Azoarcus sp gene from azotobacteria, Td gene from T4 bacteriophage, tRNALeu gene from cyanobacterium *Anabaena*, TpaCOX2, Ptu, etc.

Preferably, the spacer sequence is selected from at least one of a polyA sequence, a polyA-C sequence, a polyC sequence, or a poly-U sequence.

Preferably, the spacer sequence may be 5 to 100 nucleotides in length.

Preferably, the translation initiation element is selected from at least one of a IRES sequence, a 5' UTR sequence, a Kozak sequence, a sequence containing m6A modification (N(6) methyladenosine modification), a complementary sequence of ribosomal 18S rRNA, and an initiation sequence of rolling cycle translation.

Preferably, the coding/non-coding region can encode at least one protein selected from Gaussian luciferase (Gluc), fire fly luciferase (Fluc), enhanced green fluorescent protein (eGFP), human erythropoietin (hEPO), or Cas9 endonuclease.

Preferably, the sequence of the recombinant nucleic acid molecule is selected from at least one of SEQ ID NO: 10, SEQ ID NO: 11, SEQ ID NO: 14, SEQ ID NO: 15, or SEQ ID NO: 16.

The present invention discloses a recombinant vector comprising the recombinant nucleic acid molecule.

The present invention discloses a method for preparing circular RNA, comprising transcribing a precursor RNA using the recombinant vector, and further preparing the circular RNA from the precursor RNA.

The present invention discloses a cell comprising the recombinant vector.

The present invention discloses a composition, characterized in that, the composition comprises at least one of the recombinant nucleic acid molecule, the recombinant vector, and the cell.

Preferably, the composition further comprises one or more pharmaceutically acceptable excipients, carriers, adjuvants, or vehicles.

The present invention discloses a method for preventing, diagnosing, and/or treating diseases, and a use in the preparation of a medicament for preventing and/or treating a disease, comprising administering to a subject at least one of the flexible circularization system, the recombinant vector, the cell, the recombinant nucleic acid molecule, the recombinant vector, and the composition.

### Beneficial effects

The present invention discloses a method for constructing a vector for circular RNA preparation that is flexible and not limited by substrate sequences. Based on the research, the present inventors found that by utilizing the Azoarcus group I intron ribozyme, mutating the substrate domain sequence composed of the Exon1 (E1), P1, and Exon2 (E2) sequences of the ribozyme, while maintaining structural stability, the Azoarcus group I intron ribozyme retains enzymatic activity and the ability to form circular RNA even when the E1, P1, and E2 sequences are mutated.

Based on the research and understanding of the substrate domain of the Azoarcus group I intron, the present invention discloses a method for constructing a vector for circular RNA preparation that is flexible and not limited by substrate sequences. The steps are as follows: Step 1: Determining the target site sequence NNUNNNN to be circularized, and dividing it into E1: NNU and E2: NNNN. Step 2: Using the E1 and E2 sequences as a reference, designing an IGS sequence to form complementary pairings with E1 and E2, respectively. Step 3: Adding 5' and 3' homology arms, respectively; the target sequence to be circularized, including IRES and CDS, is linked in tandem by E2 and E1. The present invention can achieve circular RNA preparation for arbitrary target sequences without residual sequences, and with high circularization efficiency, which is a technical effect cannot be achieved by the prior art.

Based on the Azoarcus group I intron ribozyme, employing the PIE structure, various mutations are introduced into the substrate, and the mutated circularization vectors are all capable of forming circular RNA. The FlexCirc circularization system designed based on the Azoarcus group I intron ribozyme can all form circular RNA, and the circularization substrate sequences have the characteristic of flexible design. The FlexCirc circularization system designed based on the Azoarcus group I intron ribozyme has circularization efficiency of achieving a high proportion of circularization.

### Brief Description of the Drawings

Fig. 1. Panel a shows the substrate domain of the Azoarcus group I intron ribozyme composed of Exon1 (E1), P1, and Exon2 (E2) sequences, wherein E1 is the 5' end lowercase sequence cucau, E2 is the 3' end lowercase sequence aauc, P1 is the domain formed by 1-11nt of the ribozyme and E1, and IGS is the region of 6-11nt of the intron in the P1 stem-loop domain; Panel b shows a schematic diagram of a self-circularization system construct that can be flexibly designed for the FlexCirc (Flexibility self-Circularization system) circularization substrate of the present invention.
Fig. 2. A schematic diagram showing the design of variable vectors with different substrate sequences while maintaining structural stability of the substrate domain formed by E1, P1, and E2, during the preparation of circular RNA by the Azoarcus group I intron ribozyme using the permuted intron-exon (PIE) circularization system.
Fig. 3. A structural schematic diagram of the vector for circular RNA preparation using the PIE system with the Azoarcus group I intron ribozyme.
Fig. 4. Electrophoresis separation results of circularized RNA products from five circularization RNA vectors with different substrate sequences on 2% E-gel.
Fig. 5a. Electrophoresis verification results of circularization RNA vector sequences 10 and 11 after cleavage with different concentrations of RNase R, electrophoresed on 1% agarose gel.
Fig. 5b. Electrophoresis verification results of circularization RNA vector sequences 14, 15, and 16 after cleavage with different concentrations of RNase R, electrophoresed on 1% agarose gel.
Fig. 6. First-generation Sanger sequencing results after RT-PCR amplification at the circularization sites of circularization vectors with different substrates sequences 11, 14, 15, and 16.
Fig. 7. A schematic diagram of the circularization vector design for FlexCirc with different substrate sequences: sequences 17, 18, and 19.
Fig. 8. A schematic diagram of FlexCirc circular RNA formation, wherein E1-E2 can be positioned within the GOI or IRES sequence.
Fig. 9. Electrophoresis separation results on 2% E-gel of circularization products from FlexCirc vectors with different substrate sequences: sequences 17, 18, and 19.
Fig. 10. Results of 1% agarose gel electrophoresis of circularization products from FlexCirc vectors with different substrate sequences: sequences 17, 18, and 19, after cleavage with different concentrations of Rnase R.
Fig. 11. Results of 1% agarose gel electrophoresis of RT-PCR amplification products at the circularization sites of circularization products from FlexCirc vectors with different substrate sequences: sequences 17, 18, and 19.
Fig. 12. Sequencing verification results of circularization sites of circularization products from FlexCirc vectors with different substrate sequences: sequences 17, 18, and 19.
Fig. 13a. Electrophoresis separation results and purity assessment on 4% denatured PAGE gel of circularization products from FlexCirc vectors with different substrate sequences: sequences 17, 18, and 19, before and after RNase R enzyme cleavage.
Fig. 13b. Purity assessment results based on grayscale values after electrophoresis separation on 4% denatured PAGE gel of circularization products from FlexCirc vectors with different substrate sequences: sequences 17, 18, and 19, before RNase R enzyme cleavage.
Fig. 14. Verification results of eGFP fluorescent protein expression on day 3 after transfection of HEK293T cells with circularization products from FlexCirc vectors with different substrate sequences: sequences 17, 18, and 19.

### Detailed description

### Example 1: Verify the diversity of substrate core domain sequences composed of Exon1, P1, and Exon2 sequences for Azoarcus group I intron ribozyme

### Construction of AzoPIE Vector

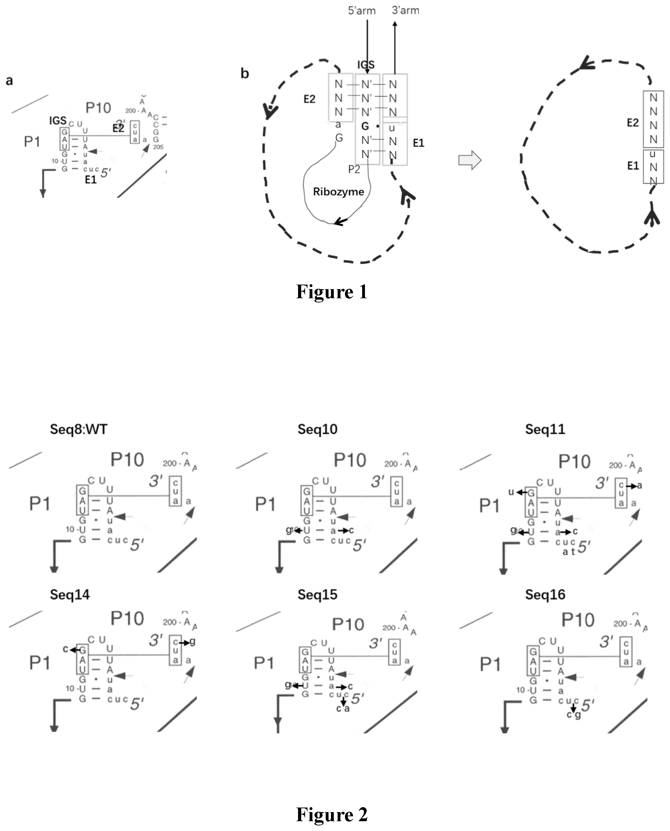

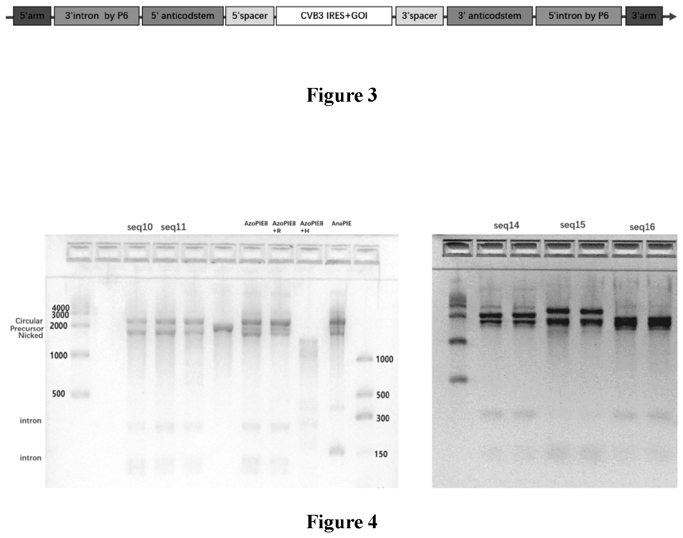

In this example, the Azoarcus group I intron ribozyme is divided from the P6 loop using the PIE strategy to form 5' half introns and 3' half introns, respectively. As shown in Figure 3, a self-circularized vector AzoPIE is constructed. Mutation vectors for the substrate core domain composed of Exon1, P1, and Exon2 are constructed. While maintaining base matching, mutation vectors for the sequences in Exon1, P1, and Exon2 are constructed respectively, as shown in Figure 2. The mutation vector sequence, comprising the 5' end T7 promoter sequence, and EcoR I sequence for DNA template linearization enzyme cleavage site, is recombined into the pBlueScript plasmid, and the desired plasmid is obtained by direct synthesis using a third-party plasmid method.

### In vitro transcription and circularization verification

### 1-2-1. Preparation of in vitro transcription DNA linearized template:

Third-party synthesized mutant vector plasmids are linearized using the restriction enzyme EcoR I to obtain transcription templates. Specifically, EcoR I restriction endonuclease is used to linearize and cleave plasmids identified as qualified by PCR to prepare IVT transcription templates. The enzyme cleavage system is shown in Table 1. HPLC method is used to detect the purity of linearized templates, ensuring that the prepared linearized templates meet the requirements for subsequent IVT transcription.

**Table 1. Formulation of 500 µL enzyme cleavage system of EcoR I enzyme**

| Compoent | Volume (µL) |
|---|---|
| Plasmid | 50µg |
| EcoR I (10U/µL) | 50 |
| Tingo Buffer | 50 |
| Nuclease-free water | Up to 500 |

### 1-2-2. In vitro transcription and circularization verification:

20 µL reaction systems are prepared according to Table 2 respectively, with two parallel experiments conducted for each sequence template. The transcription system is placed in a PCR instrument and reacted at 37°C for 2 hours. Upon completion of the reaction, 1 µL of DNase I and 2 µL of 10× DNase I buffer are added to each reaction system, followed by reaction at 37°C for 20 minutes. Each transcription system is then transferred to a 1.5 mL centrifuge tube, and 0.5 fold volumes of RNA precipitation solution containing 7.5 M lithium chloride and 50 mM EDTA are added. After mixing, the samples are precipitated at -20°C refrigerator for 1 hour. Following precipitation, the samples are centrifuged at 18360 × g at 4°C for 15 minutes, and the supernatant is removed as completely as possible.

After adding 500 µ L of 70% ethanol solution, the precipitate is rinsed and washed, centrifuged again under the same conditions. After the supernatant is again removed as completely as possible, the ethanol is evaporated to dryness, and each experimental group is resuspended in 100 µL of nuclease-free water. Samples are taken to determine RNA concentration and purity, and 2% E-Gel electrophoresis is performed to evaluate the effect of circRNA synthesis.

**Table 2. In vitro transcription system**

| **In vitro transcription**(**20 µL system**) | |
|---|---|
| **Component** | **Volume** |
| ATP/CTP/UTP/GTP(100mM) | each 2µL |
| 10×T7 buffer | 2µL |
| T7 RNA polymerase(50U/µL) | 2µL |
| RNAse inhibitor(40U/µL) | 1µL |
| Inorganic pyrophosphatase(0.1U/µL) | 2µL |
| Linear template DNA | 1µg |
| Nuclease-free water | Up to 20µL |

As shown in Figure 4, in the 2% E-gel electrophoresis separation results of 5 different mutation vector sequences, introns spontaneously cleaved by nucleases, as well as circular RNA bands and nicked RNA bands formed after circularization, are observed. Among them, the migration rate of circular RNA is slower in the 2% E-gel.

### Verification of Rnase R enzyme cleavage of Circular RNA

To further verify the presence of circular RNA in 5 different mutation vector sequences, Rnase R enzyme cleavage is performed to verify the circularization products. RNase R can cleave and degrade RNA from the 3'-5' direction, capable of digesting almost all linear RNA molecules, but difficult to digest circular RNA, lariat structures, or double-stranded RNA molecules with less than 7 nt protruding ends at the 3' end. In this example, Rnase R enzyme of different concentration units is used to perform enzyme cleavage tolerance verification. After enzyme cleavage at 37 °C for 30 minutes, 1% agarose gel electrophoresis (120V, 30 minutes) is used to observe the tolerance of circular RNA under Rnase R enzyme cleavage.

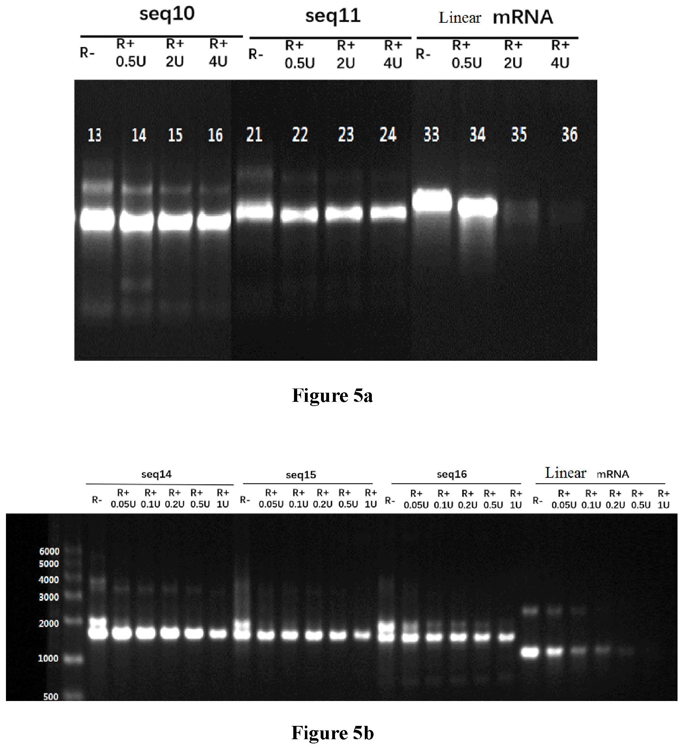

The experimental results are shown in Figures 5a and 5b: under the condition of no Rnase R enzyme cleavage, the circularization product has two obvious bands in the 1% agarose gel electrophoresis, namely linear precursor and circular RNA product bands. Under the condition of Rnase R enzyme cleavage, the linear precursor mRNA in the circularization product will be degraded, and the degradation becomes more pronounced with the increase of R enzyme concentration. Compared to linear mRNA precursors, circular RNA can tolerate different concentrations of R enzyme cleavage and has better tolerance. The corresponding linear mRNA control samples are easily degraded under R enzyme cleavage, especially at concentrations greater than 1U/L where they can be completely degraded.

### RT-PCR and Sanger sequencing verification of circularization sites of circular RNA

This example uses reverse transcription RT-PCR to amplify the circularization sites, and the amplified products are sent to a third-party sequencing company for first-generation Sanger sequencing.

**Table 3. Information on RT-PCR primers for circularization sites**

| | Primer | Primer sequence | Product size |
|---|---|---|---|
| Sequence 11 | GFP-circ-1F | CACTGGGCATGGATGAGCTG (SEQ ID NO:30) | 252bp |
| | GFP-circ-2R | GCCTGTGGGTGGGATCAAC(SEQ ID NO:31) | |
| Sequence 14 | Sequence 14-F | ACTTCTTCAAGTCGGCGATG(SEQ ID NO:32) | 372bp |
| | Sequence 14-R | CCGATTGGGTCGAAAGGTAAT (SEQ ID NO:33) | |
| Sequence 15 | Sequence 15-F | CTGGGACATAAACTGGAGTACA (SEQ ID NO:34) | 401bp |
| | Sequence 15-R | ATCAACCCACAGGCTGTTT (SEQ ID NO:35) | |
| Sequence 16 | Sequence 16-F | CCTATACTGGCTGCTTATGGTG (SEQ ID NO:36) | 400bp |
| | Sequence16-R | CATAGGCTTTGGAGCCATACA (SEQ ID NO:37) | |

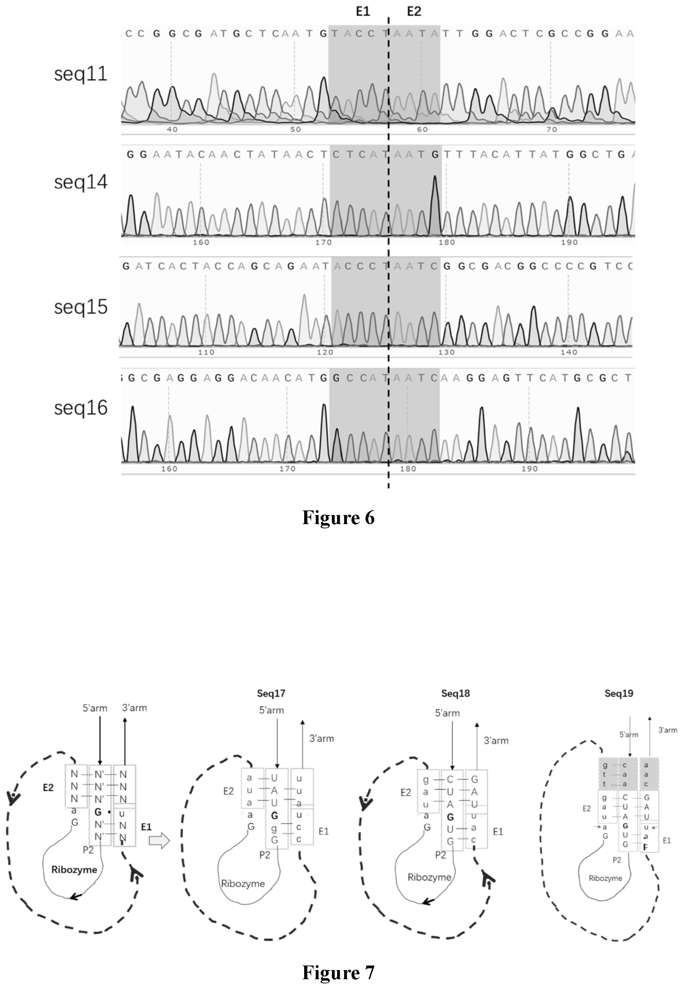

The sequencing results, as shown in Figure 6, indicate that all four different mutation vector designs can circularization, and the sequencing results of the circularization site sequences show that they can all be circularized as expected.

In this example, mutations are designed in the substrate domain (Exon1, P1, and Exon2) of the Azoarcus group I intron, and PIE circularization strategy is adopted. Circularization verification and RT-PCR sequencing of the circularization site demonstrated that different mutated substrate sequences are all capable of forming circular RNA, with ligation occurring at the expected designed site. This result indicates that the Azoarcus group I intron ribozyme, which is composed of Exon1, P1, and Exon2 as its substrate domain, possesses the characteristic of substrate sequence variability while maintaining structural stability.

### Example 2: Design and verification of FlexCirc circularization system

### 2-1. Construction of the FlexCirc system Vector

In this Example 2, the Flexible circularization (FlexCirc) system is creatively developed based on the results and implications of Example 1, namely, the substrate sequence variability of the Azoarcus group I intron.

The design method of the FlexCirc system of the present invention is described as follows, as shown in Figures 1b and 7:
Step 1: The sequence to be circularized, determined as NNUNNNN, is divided into E1: NNU and E2: NNNN.
Step 2: Using the E1 and E2 sequences as references, IGS is designed to form base complementary pairings with E1 and E2, respectively.
Step 3: 5' and 3' homologous arm sequences are added, that is, the target sequence to be circularized, including IRES, spacer, and the target protein CDS sequences.

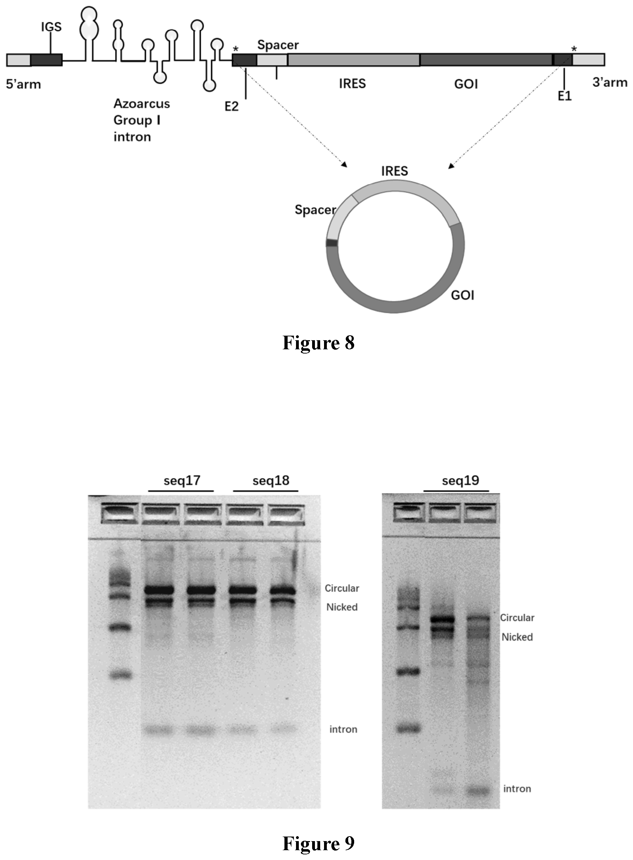

The order of the components in the FlexCirc circularization system is shown in Figure 8: a 5' homologous arm, an IGS sequence, a ribozyme, a substrate E2 sequence, a spacer sequence, an IRES element, a target protein CDS sequence, a substrate E1 sequence, and a 3' homologous arm.

The FlexCirc circularization system sequence, including the 5' end T7 promoter sequence and the DNA template linearization enzyme cleavage site EcoR I sequence, is recombined into the pBlueScript plasmid, and the desired plasmid is obtained by direct synthesis through a third-party gene synthesis company.

The three circularization substrate sequences tested in this example are listed in Table 4.

**Table 4. Information of the three substrate sequences to be circularized tested in this example**

| ID | Circularization substrate | E1 | E2 |
|---|---|---|---|
| Sequence 17 | CCTAATA (SEQ ID NO:2) | CCT (SEQ ID NO:8) | AATA(SEQ ID NO:13) |
| Sequence 18 | CATATAG (SEQ ID NO:3) | CAT (SEQ ID NO:9) | ATAG(SEQ ID NO:20) |
| Sequence 19 | CATATAGTTG (SEQ ID NO:4) | CAT (SEQ ID NO:12) | ATAGTTG (SEQ ID NO:21) |

### 2-2. In vitro transcription and circularization verification

### 2-2-1. Preparation of linearized DNA template:

The mutant vector plasmids synthesized by the third-party company are linearized by restriction endonuclease EcoR I to obtain transcription templates. Specifically, IVT transcription templates are prepared by linearization enzyme cleavage of PCR-qualified plasmids with EcoR I restriction endonuclease, and the cleavage system is shown in Table 1. The purity of the linearized templates is detected by HPLC to ensure that the prepared linearized templates meet the requirements for subsequent IVT transcription.

### 2-2-2. In vitro transcription and circularization verification:

20 µL reaction system is prepared according to Table 2 respectively, with two parallel experiments conducted for each sequence template. The transcription system is placed in a PCR instrument and reacted at 37°C for 2 hours. Upon completion of the reaction, 1 µL of DNase I and 2 µL of 10×DNase I buffer are added to each reaction system, followed by reaction at 37°C for 20 minutes. Each transcription system is then transferred to a 1.5 mL centrifuge tube, and 0.5 fold volumes of RNA precipitation solution containing 7.5 M lithium chloride and 50 mM EDTA are added. After mixing, the samples are precipitated at -20°C refrigerator for 1 hour. Following precipitation, the samples are centrifuged at 18,360 × g at 4°C for 15 minutes, and the supernatant is removed as completely as possible. After adding 500 µ L of 70% ethanol solution, the precipitate is rinsed and washed, centrifuged again under the same conditions. After the supernatant is again removed as completely as possible, the ethanol is evaporated to dryness, and each experimental group is resuspended in 100 µL of nuclease-free water. Samples are taken to determine RNA concentration and purity, and 2% E-Gel electrophoresis is performed to evaluate the effect of circRNA synthesis.

The experimental results are shown in Figure 9. In 2% E-gel electrophoresis, sequences 17, 18, and 19 are all capable of forming circRNA, nicked RNA, and released intron product band. Notably, there is only one released intron band, which differed from the two intron bands formed by the PIE circularization system, and is consistent with the design in which the Azoarcus ribozyme is not divided.

### 2-3. RNase R enzyme cleavage verification of Circular RNA

To further verify the presence of circRNA in the three different mutant vector sequences, RNase R enzyme cleavage verification is performed on the circularization products. In this example, RNase R enzymes at different concentration units are used for enzyme cleavage tolerance verification.

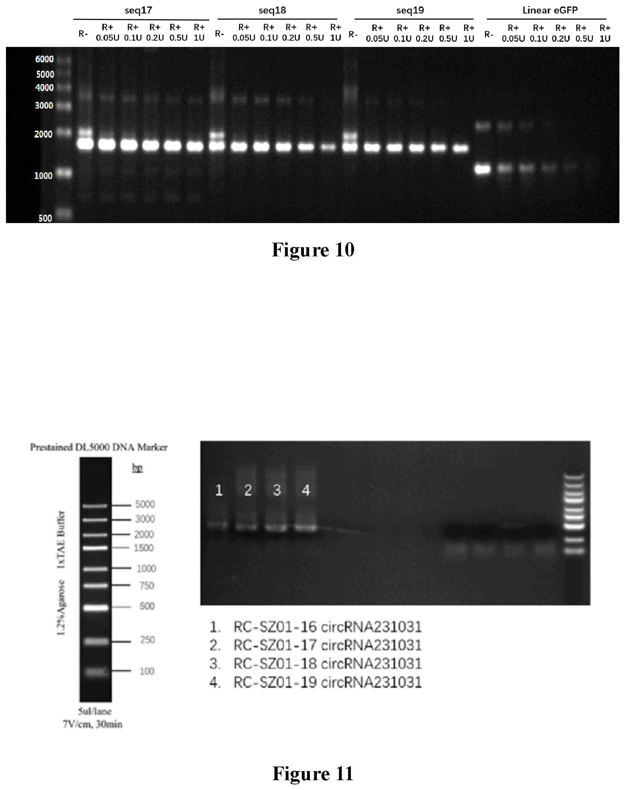

The experimental results are shown in Figure 10: Under the condition without RNase R enzyme cleavage, the circularization products exhibit two distinct bands in 1% agarose gel electrophoresis, namely the linear precursor and circRNA product bands. Under the condition with RNase R cleavage, the linear precursor mRNA in the circularization products is degraded, and the degradation becomes more pronounced with increasing R enzyme concentration. Compared with the linear mRNA precursor, circular RNA is able to tolerate enzyme cleavage by R enzyme at different concentrations, demonstrating good tolerance. The corresponding linear mRNA control samples are degraded under R enzyme cleavage, and particularly can be completely degraded at concentrations greater than 1 U/L. These results indicate that circRNA is produced in sequences 17, 18, and 19 in this example.

### 2-4. Sanger sequencing verification of circular RNA circularization sites (see Figures 10a and 10b)

In this example, reverse transcription RT-PCR is performed to amplify the circularization sites, and the amplification products are sent to a third-party sequencing company for first-generation Sanger sequencing.

**Table 5. Primer information**

| | Primer | Primer sequence | Product size |
|---|---|---|---|
| Sequence 17 | FlexCirc-F | CTGCTGCCAGACAATCACTAT(SEQ ID NO:38) | 414bp |
| | FlexCirc-R | TGTTGATCGGTGTGTGTTACTT (SEQ ID NO:39) | |
| Sequence 18 | FlexCirc-F | CTGCTGCCAGACAATCACTAT(SEQ ID NO:38) | 414bp |
| | FlexCirc-R | TGTTGATCGGTGTGTGTTACTT (SEQ ID NO:39) | |
| Sequence 19 | FlexCirc-F | CTGCTGCCAGACAATCACTAT(SEQ ID NO:38) | 414bp |
| | FlexCirc-R | TGTTGATCGGTGTGTGTTACTT (SEQ ID NO:39) | |

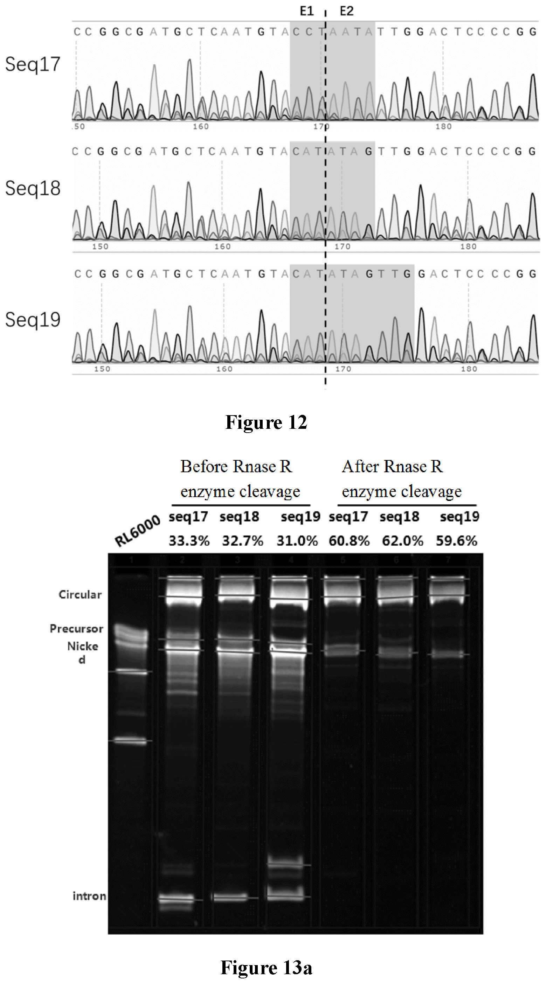

The sequencing results are shown in Figure 11: sequences 17, 18, and 19 all generate amplification products, with electrophoresis bands approximately 400 bp in size. First-generation Sanger sequencing of the amplification products is performed, and the results shown in Figure 12 demonstrated that sequences 17, 18, and 19 are all successfully circularized. Furthermore, the circularization substrate sequences of sequences 17 and 18 are different. These results indicate that the FlexCirc circularization system of the present invention could successfully prepare circular RNA for different substrate sequences. Different mutant vector designs are all capable of circularization, and sequencing of the circularization site sequences confirms that circularization occurs at the expected sites.

### 2-5. Evaluation of circularization efficiency of circular RNA

In this example, the circularization efficiency of FlexCirc circRNA is measured by 4% polyacrylamide-7 M urea denatured PAGE gel electrophoresis.

FlexCirc circularization products before and after RNase R enzyme cleavage are diluted to 80 ng/µL. Then, 5 µL of the diluted sample is mixed with 5 µL of ultrapure water and 10 µL of gel loading buffer II (1-2× solution composed of 95% formamide, 18 mM EDTA, 0.025% SDS, xylene cyanol, and bromophenol blue, Invitrogen Cat#AM8546G) at a 1:1 ratio, and pre-denatured by heating at 65°C (on a PCR instrument) for 5 min, followed by immediate cooling on ice. RNA Marker (3 µL of Marker mixed with 7 µL of ultrapure water and 10 µL of gel loading buffer II) is processed in the same manner. A 4% polyacrylamide-7 M urea denatured PAGE gel is prepared, and pre-electrophoresis is performed in 1% TBE buffer at 120 V for 30 min. The wells are rinsed 1-2 times with the buffer aspirated using a 200 µL pipette to avoid urea precipitation from the gel from affecting sample loading. Then, 15 µL (300 ng) per well is loaded (the loading amount could be adjusted appropriately, but the loading volume must be kept consistent). Electrophoresis is performed at 120 V at room temperature for 15 min, after which the voltage is increased to 200 V and electrophoresis is continued for 70 min. The gel is stained with 1:10,000 diluted SYBR Green nucleic acid dye (Thermo Fisher Scientific) for approximately 10 min, and analyzed using the Tanon 5200 chemiluminescence imaging system.

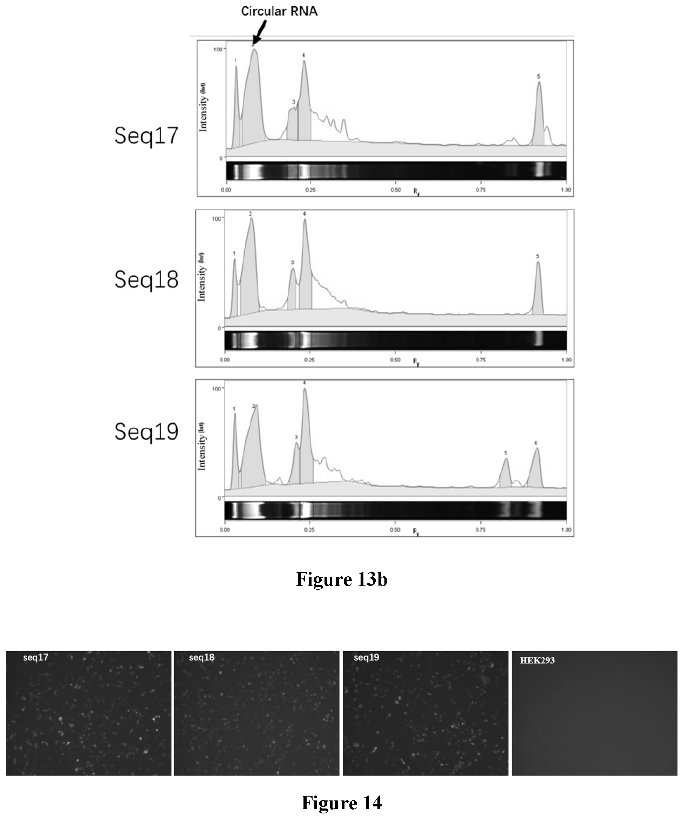

The experimental results are shown in Figure 13a: in 4% polyacrylamide-7 M urea denatured PAGE gel electrophoresis, circular RNA in the FlexCirc circularization products is located at the upper end of the gel due to its slower migration rate. Multiple different products are present in the products before RNase R enzyme cleavage, including nicked RNA, precursor RNA, and intron. After RNase R enzyme cleavage, the linear bands are all degraded and disappeared, with only circular RNA and nicked RNA retained. Quantitative analysis of the bands in each lane is performed, and the results shown in Figure 13b indicate that the pre-cleavage circularization efficiencies of sequences 17, 18, and 19 in this example are 33.3%, 32.7%, and 31.0%, respectively. The proportions of circRNA after RNase R enzyme cleavage are 60.8%, 62.0%, and 59.6%, respectively.

Sequence 19 is designed based on sequence 18 by extending the number of bases in E2 that are complementary to IGS, i.e., E2 is extended from NNNN to NNNNNNN, theoretically aiming to increase the stability of E2-IGS binding and thereby improve circularization efficiency. As shown in the experimental results in Figures 13a and 13b, extending the number of bases in E2 that are complementary to IGS does not significantly improve circularization efficiency; the circularization efficiency of sequence 19 is slightly decreased compared with sequences 17 and 18. This suggests that extending the complementary pairing between E2 and IGS cannot improve circularization efficiency, indicating that an E2 sequence of NNNN is optimal.

### 2-6. Verification of circular RNA protein expression

### Cell culture

A bottle of HEK293T/17 cells cultured with T75 is taken and when the cell confluence reached 80%-90%, it is plated. The culture medium is discarded, and the cells are washed with PBS, after which the PBS is removed. The cells are then digested with 0.05% trypsin until they became rounded, the trypsin is discarded, and the cells are dispersed by pipetting with 10 mL of 10% FBS culture medium without antibiotics. Following cell counting, the cell concentration is adjusted to 1×10⁶ cells/mL ± 10%. Ten 48-well plates are prepared, and 0.2 mL of cell suspension is inoculated into each plate. The cell culture plates are then placed in a 37°C, 5% CO₂ incubator and cultured for 24 hours ± 4 hours. 0.4 µg of circRNA is dissolved in 25 µL of serum-free DMEM medium, gently mixed, and placed at room temperature for 5 minutes. 1 µL of Lipohigh transfection reagent is dissolved in 25 µL of serum-free DMEM medium, gently mixed, and placed at room temperature for 5 minutes. The medium containing LipoHigh is then added to the medium containing circRNA, gently mixed, and placed at room temperature for 20 minutes. The cell culture plates are removed from the incubator, and the transfection mixture is added dropwise to the inoculated cells. After gentle mixing, the plates are placed in a 37°C, 5% CO₂ incubator for culture. After 4-8 hours, the solution is discarded and replaced with 0.5 mL of medium containing 2% FBS for continued culture.

### Cell culture and fluorescence detection

The GFP fluorescence expression is observed and photographed under a fluorescence microscope with a 10× eyepiece and 10× objective lens using one 48-well plate, after transfection for 24 hours ± 4 hours. After taking the photo, a multifunctional microplate reader is used to detect the fluorescence intensity value. From the fourth day to the twelfth day, the detection is carried out according to the operation of the third day, and the process of fluorescence from zero to peak and from peak to decrease in these days is monitored.

The experimental results are shown in Figure 14. On day 3 after transfection of HEK293T/17 cells with circular RNAs of sequence 17, sequence 18, and sequence 19 that had been purified by RNase R enzyme cleavage, green fluorescence is observed in all cases, indicating normal expression of the eGFP protein. This demonstrates that the circular RNAs prepared using the FlexCirc circRNA is capable of protein expression.

## Claims

1. A flexible circularization (FlexCirc) system, **characterized in that** the system comprises: a 5' homologous arm, an IGS sequence, a ribozyme, a substrate E2 sequence, a spacer sequence, an IRES element, a target protein CDS sequence, a substrate E1 sequence, and a 3' homologous arm.

2. The flexible circularization system of claim 1, **characterized in that** the system comprises following elements in sequence from 5' end to 3' end: the 5' homologous arm, the IGS sequence, the ribozyme, the substrate E2 sequence, the spacer sequence, the IRES element, the target protein CDS sequence, the substrate E1 sequence, and the 3' homologous arm.

3. The flexible circularization system of any one of claims 1-2, **characterized in that** the system further comprises a T7 promoter sequence at the 5' end and a DNA linearization enzyme cleavage site.

4. The flexible circularization system of claim 3, **characterized in that** the enzyme cleavage site is EcoR I.

5. The flexible circularization system of any one of claims 1-2, **characterized in that** the substrate E1 sequence is selected from SEQ ID NO: 8, SEQ ID NO: 9, or SEQ ID NO: 12; the substrate E2 sequence is selected from SEQ ID NO: 13, SEQ ID NO: 20, or SEQ ID NO: 21.

6. The flexible circularization system of any one of claims 1-2, **characterized in that** a sequence of the 5' homologous arm is shown as SEQ ID NO: 1.

7. The flexible circularization system of any one of claims 1-2, **characterized in that** the IGS sequence is selected from SEQ ID NO: 5, SEQ ID NO: 6, or SEQ ID NO: 7.

8. The flexible circularization system of any one of claims 1-2, **characterized in that** the ribozyme sequence is shown as SEQ ID NO: 22.

9. The flexible circularization system of any one of claims 1-2, **characterized in that** the spacer sequence is selected from SEQ ID NO: 23 or SEQ ID NO: 26.

10. The flexible circularization system of any one of claims 1-2, **characterized in that** the IRES element sequence is shown as SEQ ID NO: 24.

11. The flexible circularization system of any one of claims 1-2, **characterized in that** the target protein CDS sequence is shown as SEQ ID NO: 25.

12. The flexible circularization system of any one of claims 1-2, **characterized in that** a sequence of the 3' homologous arm is selected from SEQ ID NO: 27, SEQ ID NO: 28, or SEQ ID NO: 29.

13. The flexible circularization system of any one of claims 1-2, **characterized in that** the circularization system is a recombinant nucleic acid molecule, and a sequence of the recombinant nucleic acid molecule is shown as SEQ ID NO: 17, SEQ ID NO: 18, or SEQ ID NO: 19.

14. A recombinant vector, **characterized in that** the recombinant vector comprises the flexible circularization system of any one of claims 1-13.

15. The recombinant vector of claim 14, **characterized in that** the recombinant vector is pBlueScript.

16. A cell, **characterized in that** the cell comprises the flexible circularization system of any one of claims 1-13 or the recombinant vector of any one of claims 14-15.

17. A method for preparing circular RNA, **characterized in that** the method comprises transcribing a precursor RNA using the recombinant vector of any one of claims 14-15, and further preparing the circular RNA from the precursor RNA.

18. A composition, **characterized in that** the composition comprises at least one of the flexible circularization system of any one of claims 1-13, the recombinant vector of any one of claims 14-15, and the cell of claim 16.

19. A design method for a flexible circularization (FlexCirc) system, **characterized in that** the design method comprises the following steps:
1) determining a site sequence NNUNNNN to be cyclized, dividing the site sequence into E1: NNU, and E2: NNNN, wherein N is any nucleotide;
2) Using the E1 and E2 sequences as references to design an IGS sequence to form complementary base pairings with E1 and E2, respectively;
3) further adding 5' and 3' homologous arm sequences, a ribozyme sequence, IRES, a spacer sequence and a target protein CDS sequence, respectively.

20. The method of claim 19, **characterized in that**, in step 3), the order of each element from 5' to 3' ends is the 5' homologous arm, the IGS sequence, the ribozyme, the substrate E2 sequence, the spacer sequence, the IRES element, the target protein CDS sequence, the substrate E1 sequence, and the 3' homologous arm.

21. The method of any one of claims 19-20, **characterized in that**, the method further comprises:
4) introducing a T7 promoter sequence at the 5' end and a DNA template linearization enzyme cleavage site into the flexible circularization system.

22. The method of claim 21, **characterized in that**, the enzyme cleavage site is EcoR I.

23. A recombinant nucleic acid molecule, **characterized in that**, the recombinant nucleic acid molecule comprises a 5' homologous arm, a first intron, a substrate enhancing element, a spacer sequence, a translation initiation element, a coding/non-coding region, a spacer sequence, a substrate enhancing element, a second intron, and a 3' homologous arm.

24. The recombinant nucleic acid molecule of claim 23, **characterized in that**, the recombinant nucleic acid molecule comprises the 5' homologous arm, the first intron, the substrate enhancing element, the spacer sequence, the translation initiation element, the coding/non-coding region, the spacer sequence, the substrate enhancing element, the second intron, and the 3' homologous arm in sequence from 5' end to 3' end.

25. The recombinant nucleic acid molecule of claim 24, **characterized in that**, a fragment of the intron is preferably a type I intron fragment, and more preferably, an Azoarcus group I intron; further, the type I intron is derivable from any of following type I introns: Azoarcus sp gene from azotobacteria, Td gene from T4 bacteriophage, tRNALeu gene from cyanobacterium *Anabaena*, TpaCOX2, Ptu, etc.

26. The recombinant nucleic acid molecule of any one of claims 23-24, **characterized in that**, the spacer sequence is selected from at least one of polyA sequence, polyA-C sequence, polyC sequence, or poly-U sequence.

27. The recombinant nucleic acid molecule of claim 26, **characterized in that**, the spacer can be 5 to 100 nucleotides in length.

28. The recombinant nucleic acid molecule of any one of claims 23-24, **characterized in that**, the translation initiation element is selected from at least one of a IRES sequence, a 5' UTR sequence, a Kozak sequence, a sequence containing m6A modification (N(6) methyladenosine modification), a complementary sequence of ribosomal 18S rRNA, and an initiation sequence of rolling circle translation.

29. The recombinant nucleic acid molecule of any one of claims 23-24, **characterized in that**, the coding/non-coding region can encode at least one protein selected from Gaussian luciferase (Gluc), fire fly luciferase (Fluc), enhanced green fluorescent protein (eGFP), human erythropoietin (hEPO), or Cas9 endonuclease.

30. The recombinant nucleic acid molecule of any one of claims 23-24, **characterized in that**, a sequence of the recombinant nucleic acid molecule is selected from at least one of SEQ ID NO: 10, SEQ ID NO: 11, SEQ ID NO: 14, SEQ ID NO: 15, or SEQ ID NO: 16.

31. A recombinant vector comprising the recombinant nucleic acid molecule of any one of claims 23-30.

32. A method for preparing circular RNA, comprising transcribing a precursor RNA using the recombinant vector of claim 31, and further preparing the circular RNA from the precursor RNA.

33. A cell comprising the recombinant vector of claim 31.

34. A composition, **characterized in that**, the composition comprises at least one of the recombinant nucleic acid molecule of any one of claims 23-30, the recombinant vector of claim 31, and the cell of claim 33.

35. The composition of claim 34, further comprising one or more pharmaceutically acceptable excipients, carriers, adjuvants, and vehicles.

36. A method for preventing, diagnosing, and/or treating diseases, comprising administering to a subject at least one of the flexible circularization system of any one of claims 1-13, the recombinant vector of any one of claims 14-15, the cell of claim 16, the recombinant nucleic acid molecule of any one of claims 23-30, the recombinant vector of claim 31, the cell of claim 33, and the composition of any one of claims 34-35.
